# FASCICULE DE BREVET EUROPEEN

(11) **EP 1 525 210 B1**
(45) Date de publication et mention de la délivrance du brevet: **23.05.2012**
(21) Numéro de dépôt: 03750834.8
(22) Date de dépôt: 22.07.2003
(51) Int. Cl.: C07H 21/00

(54) **PROCEDE DE FABRICATION DE PUCES BIOLOGIQUES**
VERFAHREN ZUR HERSTELLUNG VON BIOCHIPS
METHOD FOR MAKING BIOCHIPS

(30) Priorité: 25.07.2002 FR 0209456
(43) Date de publication de la demande: 27.04.2005
(73) Titulaire: CENTRE NATIONAL DE LA RECHERCHE SCIENTIFIQUE (CNRS), 75016 Paris (FR); UNIVERSITE DE NANTES, 44035 Nantes (FR); University of Florida, Gainesville, FL 32611-5500 (US)
(72) Inventeur: TELLIER, Charles, F-44130 NOTRE DAME DES LANDES (FR); PIPELIER, Muriel, F-44300 NANTES (FR); DUBREUIL, Didier, F-44710 PORT SAINT PERE (FR); BUJOLI, Bruno, F-44240 SUCE SUR ERDRE (FR); TALHAM, Daniel, GAINESVILLE, FL 32601 (US)
(74) Mandataire: Blot, Philippe Robert Emile
(86) Numéro de dépôt international: PCT/FR2003/002318
(87) Numéro de publication internationale: WO 2004/011401

(56) Documents cités:
- BELLEZZA F. ET AL.: "Zirconium phosphate and modified zirconium phosphates as supports of lipase. Preparation of the composites and activity of the supported enzyme." LANGMUIR, vol. 18, 2002, pages 8737-8742, XP002240673
- KUMAR C V ET AL: "Proteins immobilized at the galleries of layered [alpha]-zirconium phosphate: Structure and activity studies" JOURNAL OF THE AMERICAN CHEMICAL SOCIETY 09 FEB 2000 UNITED STATES, vol. 122, no. 5, 9 février 2000 (2000-02-09), pages 830-837, XP002266758 ISSN: 0002-7863
- XU X H ET AL: "IMMOBILIZATION OF DNA ON AN ALUMINUM(III) ALKANEBISPHOSPHONATE THIN FILM WITH ELECTROGENERATED CHEMILUMENESCENT DETECTION" JOURNAL OF THE AMERICAN CHEMICAL SOCIETY, AMERICAN CHEMICAL SOCIETY, WASHINGTON, DC, US, vol. 116, 1994, pages 8386-8387, XP000929137 ISSN: 0002-7863
- BENÍTEZ I. O. ET AL: "Monolayers as models for supported catalysts: zirconium phosphonate films containing manganese(III) porphyrins" J. AM. CHEM. SOC., vol. 124, 2002, pages 4363-4370, XP002240672 cité dans la demande
- DATABASE WPI Section Ch, Week 200170 Derwent Publications Ltd., London, GB; Class B04, AN 2001-608992 XP002240674 & JP 2001 178470 A (FUJI PHOTO FILM CO LTD), 3 juillet 2001 (2001-07-03)
- IHARA T. ET AL.: "DNA separation using Zr(IV)-loaded resin through ligand exchange" ANALYTICAL SCIENCES, vol. 17, 2001, page i1229 XP008016581

## Description

L'invention concerne un procédé d'immobilisation d'ADN, de protéines ou d'oligo ou polysaccharides sur un support solide pour la fabrication de puces biologiques, et les puces obtenues par ce procédé.

Différents procédés sont aujourd'hui utilisés pour la fabrication de puces à ADN. Selon une première méthode, des oligonucléotides sont synthétisés directement sur le support après ancrage covalent d'un précurseur (système Affymetrix, Niemeyer et Blohm Angew ; Chem. Int. Ed. 1999, 38, 2865-2869), ce qui conduit à des puces de coût élevé. Le fournisseur ne fournit en outre pas tous les détails sur les séquences des oligonucléotides immobilisés.

Selon une deuxième approche, les oligonucléotides sont présynthétisés, biotinylés et déposés ensuite sur une plaque activée par une couche de streptavidine. L'accrochage se fait via des interactions spécifiques non covalentes biotine-streptavidine, cette dernière étant une protéine ayant l'inconvénient d'être de stabilité modérée. Lorsque les puces sont constituées de fragments d'ADNc, ceux-ci sont le plus souvent immobilisés par adsorption sur une couche d'aminosilane ou de polylysine. Cette dernière stratégie pose souvent des problèmes de répétabilité et d'homogénéité de surface liés à la difficulté de contrôler la qualité du film de polylysine et sa stabilité.

Selon une troisième voie, les oligonucléotides ou ADNc sont ancrés sur le support grâce à un couplage covalent, via un lien chimique organique. Cela nécessite (a) la fonctionnalisation de la surface par des chaînes à terminaisons réactives, par exemple des fonctions acide carboxylique activées, (b) la modification des oligonucléotides ou ADNc en position 5' par une fonction, par exemple une amine primaire, susceptible de réagir avec les terminaisons mentionnées ci-dessus. Il s'agit de produits "clés en main" d'un coût relativement élevé, imposant l'achat des oligonucléotides convenablement modifiés. Ces approches sont décrites notamment dans la demande de brevet WO 01/42 495.

La technologie des puces à ADN est également discutée dans l'article de revue de Wang, Nucleic Acids Res., 2000, 28(16):3011-3016.

Parallèlement, ont été développées des puces sur lesquelles des polymères biologiques autres que de l'ADN étaient immobilisés, par exemple des protéines ou peptides.

Cette technologie a été mise au point principalement par adaptation de la technologie des puces à ADN (cf par exemple la demande WO 93/22 680 d'Affymax).

Une approche de fixation de protéines par liaison covalente est par exemple rapportée dans Mac Beath et Schreiber, Science, 2000, 289:1760-1763, Mitchell, Nature Biotechnology, 2002, 20, 225-229 et « Protein microarray technology », dans Trends in Biotechnology, 2002, 20(4), 160-166.

Les auteurs de la présente invention ont maintenant mis au point un nouveau procédé de fabrication de produits de type puces biologiques, qui présente en outre des avantages vis-à-vis des techniques existantes.

Il s'agit d'immobiliser des polymères biologiques, généralement de séquence connue, tels qu'ADN, protéines, oligo ou polysaccharides, porteurs d'un groupement phosphate (OP(O)(OH)₂) libre, sur un support solide présentant une surface recouverte d'un métal capable de liaison de coordination avec lesdits groupements phosphate tel que, de préférence, un support solide présentant en surface une couche de phosphonate métallique. Les groupements phosphate, qui servent de fonctions d'ancrage peuvent être présents naturellement dans le polymère ou introduits par modification enzymatique ou chimique.

L'ancrage s'effectue par liaison iono-covalente entre le groupement phosphate libre du polymère et le métal.

Ce mode d'ancrage, du type iono-covalent, est plus résistant que les systèmes mettant en jeu des interactions du type liaison hydrogène ou du type électrostatique (comme c'est le cas avec la polylysine par exemple).

La présente invention a donc pour objet une méthode de fabrication d'un produit de type puce biologique comprenant l'immobilisation d'au moins un polymère biologique portant un groupement phosphate libre sur un support solide présentant une surface recouverte d'un métal capable de liaison de coordination avec un groupement phosphate. Par "support solide présentant une surface recouverte d'un métal", on entend, en général, un support présentant en surface un métal, de préférence présent sous forme d'une monocouche métallique. De préférence, ce support est un support solide revêtu d'une couche d'un phosphonate métallique. Selon le procédé de l'invention, le polymère biologique est immobilisé sur la surface du support par liaison iono-covalente entre le groupement phosphate libre dudit polymère et le métal accessible en surface.

Un autre objet de l'invention est un produit obtenu par ce procédé, à savoir un produit de type puce biologique, comprenant un support solide plan présentant une surface recouverte d'un métal capable de liaison de coordination avec un groupement phosphate, au moins un polymère biologique portant un groupement phosphate étant immobilisé sur ladite surface par liaison iono-covalente.

Un kit de préparation d'un produit tel que défini précédemment fait également partie de l'invention. Ce kit comprend les éléments suivants :
- un support solide présentant une surface recouverte d'un métal capable de liaison de coordination avec un groupement phosphate ;
- au moins un polymère biologique portant un groupement phosphate libre ;
- éventuellement des réactifs. Une solution d'acide phosphonique peut par exemple être utile, dans l'hypothèse où on désirerait saturer les sites de coordination sur les zones non ciblées. Une solution d'acide phosphonique 1 mM (exemple : C₄H₉PO₃H₂) peut notamment être utilisée. Avantageusement, une solution d'albumine de sérum bovin (BSA "Bovine Serum Albumina") peut également être utilisée (typiquement une solution à 1 % en masse de BSA, 3% de SDS dans le SSC 3,5X).

L'invention vise par ailleurs l'utilisation d'un produit de type puce biologique tel que défini ci-dessus, à des fins de criblage de composés susceptibles de se lier audit polymère biologique immobilisé, ou comme outil de diagnostic *in vitro.*

### Préparation du polymère biologique

Par "polymère biologique", on entend un composé à base d'unités monomériques reliées entre elles, le plus souvent selon une séquence connue, et qui présente une activité biologique ou réagit avec un composé ayant une activité biologique.

On peut citer notamment les acides nucléiques, tels que ARN ou ADN, en particulier de l'ADNc ou des oligonucléotides ; des peptides, protéines, oligo ou polysaccharides. Des monomères synthétiques, qui n'existent pas de manière naturelle, peuvent éventuellement être utilisés pour construire un polymère biologique. Dans cette optique, peuvent par exemple être utilisés des carbamates, phosphonates, sulfonamides, et sulfoxydes.

Comme autres exemples de polymère biologique, on peut également citer les PNA ("peptide nucleic acid") et les aptamers.

De manière générale, on peut immobiliser des oligonucléotides, oligoribonucléotides ou peptides que l'on peut former par des techniques combinatoires et qui sont doués de propriétés de reconnaissance vis-à-vis de molécules d'intérêt à détecter.

De manière avantageuse, on peut préparer ou obtenir des polymères biologiques qui présentent un groupement espaceur entre le groupement phosphate et le corps du polymère. Dans le cas où le polymère est un acide nucléique, on peut utiliser notamment des espaceurs du type polyA, polyC, polyT ou polyG (généralement d'environ 10 mer, et par exemple de 7 à 9 mer), dont l'introduction via un synthétiseur est peu onéreuse. De préférence, lorsque le polymère est un acide nucléique, tel que l'ADN, le groupement espaceur est un motif polyguanine polyG (à savoir un enchaînement d'unités guanine, et de préférence un enchaînement de 7 à 9 unités guanine).

De manière préférentielle, le polymère biologique est un acide nucléique, avantageusement de l'ADN, phosphorylé (à savoir porteur d'un groupement phosphate OP(O)(OH)₂) en position 5', le groupement phosphate étant de préférence séparé du corps de l'acide nucléique par un groupement polyguanine (polyG).

Cette phosphorylation peut être réalisée facilement au moyen d'enzymes de type kinases, par exemple la T4 polynucléotide kinase en présence d'ATP, classiquement utilisée dans les réactions de PCR.

Le polymère biologique peut également être un acide nucléique, par exemple de l'ADN, phosphorylé (à savoir porteur d'un groupement OP(O)(OH)₂) en position 3', le groupement phosphate étant de préférence séparé du corps de l'acide nucléique par un groupement polyguanine (polyG). La phosphorylation en position 3' peut être réalisée chimiquement par des techniques standards, relatives à la chimie des phosphoramidites.

Les acides nucléiques utilisés sont de préférence constitués de 25 à 70 mer (c'est-à-dire paires de bases), de préférence 40 - 50 mer, pour les oligonucléotides et de 100 à 2000 paires de bases pour les ADNc.

Selon un autre mode de réalisation, le polymère biologique est une protéine, un oligo ou polysaccharide ou un peptide fonctionnalisé ou modifié par un groupement phosphate. Cette modification peut être réalisée par des méthodes chimiques ou enzymatiques, sauf bien sûr dans le cas où des groupements phosphate libres sont présents naturellement, comme c'est le cas pour des peptides, protéines, voire même des oligo- ou polysaccharides déjà phosphorylés.

### Préparation du support

Le support solide plan choisi peut être de n'importe quelle matière appropriée pour la fabrication de produits de type puces selon l'invention. On peut utiliser notamment un support en verre, en silicium, en mica, en quartz, en plastique, ou à base de divers polymères synthétiques. Le support peut être en outre recouvert d'une fine couche d'or. Les supports en verre sont préférés, et on peut utiliser par exemple une lame de microscope, plus généralement toute plaque ou lame de verre, de quartz ou de silicium. La forme du support n'a pas d'importance.

Conformément à l'invention, le support présente une surface recouverte d'un métal, en général sous forme cationique. Ce métal est choisi de manière à être capable de liaison de coordination avec un groupement phosphate. Le zirconium est particulièrement adapté, mais on peut citer également, à titre d'exemples, d'autres métaux tétravalents comme le titane, le vanadium, l'étain ; des métaux trivalents comme l'aluminium, le fer, le chrome, le gallium ; toute une série de métaux divalents comme le zinc, le manganèse, le cuivre, le cobalt, le nickel ; quelques cas de métaux hexavalents comme le molybdène, l'uranium, le tungstène. Une revue récente sur les métallophosphonates récapitule ces possibilités (A. Clearfield in Progress in Inorganic Chemistry, Vol. 47, (Ed.: K. D. Karlin), John Wiley & Sons, Inc., New York, 1998, pp. 371-510).

De préférence, ce métal est déposé sur la surface du support sous la forme d'une monocouche, de préférence une monocouche d'un phosphonate dudit métal.

Ainsi, le métal peut notamment être lié à la surface du support par l'intermédiaire d'une molécule ou bras espaceur. Il peut s'agir par exemple d'une chaîne d'acide gras portant un groupement phosphonate (par exemple, l'acide octadécylphosphonique), auquel se lie le métal par liaison iono-covalente.

Selon un mode de réalisation particulier de l'invention, la molécule espaceur est l'acide octadécylphosphonique et le métal est le zirconium.

De manière avantageuse, on utilise des lames de verre sur lesquelles repose un film de type Langmuir-Blodgett à base de phosphonate de zirconium, préparées par le procédé tel que décrit dans Benitez et al., J. Am. Chem. Soc., 2002, 124:4363-4370, par immersion dans différentes solutions aqueuses. Dans ce cas, le film est lié au support de verre via des interactions hydrophobe-hydrophobe.

Ce procédé met en oeuvre de l'acide octadécylphosphonique qui est une molécule amphiphile qui, une fois déposée à la surface d'une phase aqueuse, oriente sa tête polaire PO₃H₂, côté eau et sa chaîne carbonée côté air. En comprimant ces molécules, on peut alors former une monocouche de Langmuir-Blodgett qui peut être transférée sur une lame de verre ayant subi un traitement pour la rendre hydrophobe (par exemple, un traitement avec l'octadécylchlorosilane). A ce stade, la lame est devenue hydrophile puisque ce sont les groupes acide phosphonique qui sont présents à la surface. Ceux-ci ont une forte affinité pour les ions métalliques tels que le zirconium pour former des liaisons iono-covalentes métal-PO₃.

On obtient ainsi une surface à caractère métallique, constituée d'atomes de zirconium arrangés de manière régulière, en général sous la forme d'une monocouche, la couche Langmuir-Blodgett mesurant alors 2,4 nm d'épaisseur.

Les supports mis en oeuvre conformément à l'invention ont l'avantage d'être stables au cours du temps, et ne nécessitent pas de pré-activation nécessaire. Ils peuvent par ailleurs être stockés simplement dans l'eau.

Le film de phosphonate de zirconium peut également être fixé de façon covalente au support, en procédant comme décrit dans Katz et al., Chem. Mater., 1991, 3:699-703. Une première possibilité est de fonctionnaliser la surface de verre par le 3-aminopropyltriméthoxysilane qui vient se greffer sur la surface, et les fonctions NH₂ terminales sont ensuite traitées par POCl₃ et une base, pour introduire en bout de chaîne les groupes PO₃H₂ qui sont prêts à recevoir la couche de zirconium. Une autre possibilité est de prendre des lames de mica ou de silicium recouvertes d'une couche d'or, et de les traiter avec l'acide 8-mercaptooctylphosphonique (HS-(CH₂)₈-PO₃H₂) qui se greffe via un couplage thiol / or.

Des lames de borosilicate revêtues d'une fine couche d'or peuvent également être fonctionnalisées par des terminaisons PO₃H₂ selon Brochsztain et al, J. Mater. Chem., 2002, 12:1250-1255, par traitement successivement avec du 2-mercaptoéthanol (HS-C₂H₅-OH) et une solution (POCl₃ + collidine).

### Immobilisation des polymères biologiques

Les polymères biologiques peuvent être déposés sur le support par simple "spotting". De manière usuelle, il s'agit d'une opération de dépôt de microgouttes (par exemple environ 50 picolitres) ou « spots », au moyen d'un robot effectuant des prélèvements dans les puits de plaques de microtitration, par exemple.

Les polymères se fixent par liaison de coordination, de type iono-covalente, avec le métal à la surface du support.

Les spots sont de préférence arrangés sous la forme d'un réseau (ou "array") organisé. Tel qu'utilisé ici, le terme réseau ou "array" est un arrangement ordonné de spots de polymères biologiques, comme dans une matrice de lignes ou de colonnes. Typiquement, pour des spots d'environ 150 microns de diamètre et espacés de 300 microns, la densité de spots est de l'ordre de 500 par cm². De préférence, le réseau contient plus d'un polymère biologique immobilisé.

Dans le contexte de la présente invention, le terme "produit ou dispositif de type puce biologique" doit être compris comme incluant tout support solide sur lequel au moins un polymère biologique est immobilisé.

Du fait de l'orientation des polymères biologiques substantiellement perpendiculaire au support, on peut éventuellement immobiliser ces polymères à une haute densité, notamment jusqu'à une densité de 10¹⁰-10¹² polymères par cm².

Un objet particulier de l'invention est un produit de type puce biologique, comprenant une lame de verre présentant une surface recouverte d'une monocouche d'octadécylphosphonate de zirconium, au moins un acide nucléique portant un groupement phosphate en 5' étant immobilisé sur ladite surface par liaison iono-covalente entre ledit groupement phosphate de l'acide nucléique et le zirconium.

### Utilisation des produits fabriqués

Les produits de type puce biologique fabriqués conformément à l'invention trouvent de multiples applications, par exemple pour des analyses biologiques et du criblage de composés susceptibles de se lier aux polymères immobilisés.

De manière générale, l'analyse des puces et "arrays" peut être réalisée selon diverses techniques bien connues de l'homme du métier (par exemple fluorescence, radioactivité, spectrométrie de masse, résonance plasmonique de surface, infra-rouge, chimiluminescence).

Lorsque le polymère biologique est un acide nucléique, les produits de l'invention constituent des outils performants pour l'analyse en parallèle d'un grand nombre de gènes ou de séquences d'ADN ou d'ARN. Leur principe de fonctionnement repose sur la propriété d'hybridation ou d'appariement de deux brins de séquences complémentaires afin de reconstituer la double hélice d'ADN. Selon un mode de réalisation particulier, des sondes d'oligonucléotides de séquence connue, immobilisées sur un substrat support, sont mises en présence de cibles extraites d'un échantillon biologique à analyser, et marquées à l'aide de marqueurs fluorescents.

Selon un mode de réalisation particulier, pour savoir où il y a eu hybridation, la puce est ensuite lue sur un microscope confocal, puis analysée en quantifiant l'intensité de fluorescence sur les différents spots, chacun d'eux correspondant à une séquence donnée.

Les produits de l'invention sont particulièrement adaptés pour étudier des profils d'expression d'ARNm, séquences des acides nucléiques, ou rechercher des polymorphismes ou des mutations dans de l'ADN génomique, par exemple.

De manière générale, les produits de l'invention constituent des outils diagnostiques simples et pratiques d'utilisation, pour la détection de maladies infectieuses ou génétiques notamment.

Les figures et exemples suivants illustrent l'invention sans en limiter la portée.

### LEGENDE DES FIGURES :

La figure 1 représente un schéma de principe de formation de films Langmuir-Blodgett (LB) à partir d'acides phosphoniques à chaîne longue. Le dépôt du film LB a lieu sur les deux faces de la lame de verre.

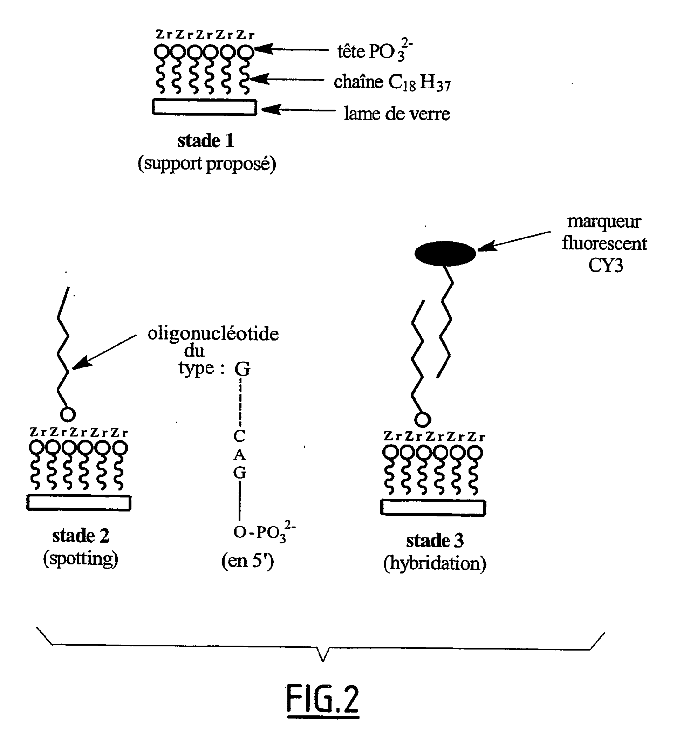

La figure 2 représente un schéma d'ancrage d'un oligonucléotide sur une plaque portant une couche de phosphonate de zirconium, et l'utilisation du produit obtenu par un test d'hybridation.
La figure 3 est un diagramme représentant l'intensité de la fluorescence observée lors des tests réalisés dans les conditions de l'exemple 3 décrit ci-après.

### EXEMPLES

### Exemple 1 : Immobilisation d'oligonucléotides

### 1.1 Obtention du support

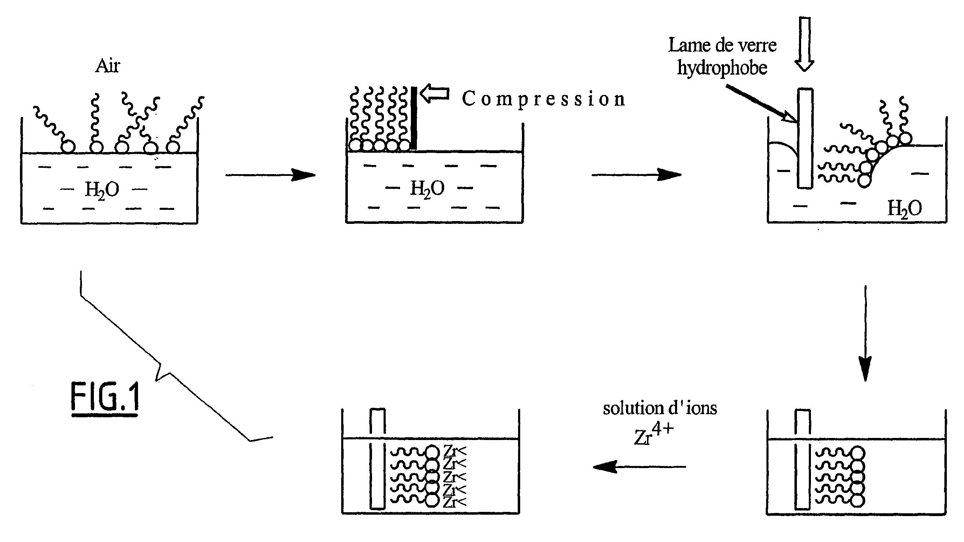

On utilise des lames de verre revêtues d'un film LB à base de phosphonate de zirconium, comme illustré à la figure 1 et comme décrit dans Benitez et al., J. Am. Chem. Soc., 2002, 124:4363-4370.

Les lames de verre sont ensuite stockées dans de l'eau ultrapure (résistivité voisine de 18 MΩ/cm). Avant utilisation, celles-ci sont séchées par centrifugation avant d'être spottées à l'aide d'un robot. Les deux côtés de la lame peuvent être utilisés indifféremment.

### 1.2 Essai préliminaire :

Une première étude de faisabilité a montré qu'un oligonucléotide de 35 bases, phosphorylé en position 3' et marqué en position 5' par une sonde fluorescente CY3, s'ancre spontanément par simple "spotting" sur les films de Langmuir-Blodgett à base de phosphonate de zirconium. Il apparaît que c'est bien le phosphate terminal qui est responsable de l'ancrage sur le film, car pour un oligonucléotide identique non phosphorylé en 3', dans les mêmes conditions dé "spotting", la quantité de' sondes fixée est très inférieure (d'un facteur 5). Cette fixation "non spécifique" est probablement provoquée par une interaction du zirconium avec les groupements phosphodiester joignant les unités nucléotidiques. De plus, la fixation est stable dans les conditions standard de lavage des puces à ADN. On constate donc un bon accrochage de l'oligonucléotide phosphorylé par rapport à l'homologue non phosphorylé.

### 1.3 Oligonucléotides employés :

On a immobilisé quatre types d'oligonucléotides, à savoir :
   - un oligonucléotide 35 mer (baptisé 1);
   - son analogue phosphorylé en position 5' (baptisé 2 = 1-5'-OPO₃H₂);
   - l'analogue de 2 comportant un groupe espaceur de 11 motifs adénine, entre l'oligonucléotide et le groupement phosphate terminal (baptisé 3 = 1-(A)₁₁-5'-OPO₃H₂); et
   - l'analogue de 3 non phosphorylé (baptisé 4 = 1-(A)₁₁).

### 1.4 Immobilisation des oligonucléotides :

Les oligonucléotides en solution dans du SSC 1X (pH ajusté à 6 par ajout d'HCl) ont été "spottés" sur ces lames, à des concentrations de 50 µM, 20 µM et 5 µM.

Après "spotting", on laisse sécher les lames à l'air libre et on les laisse pendant 24 heures dans une boite fermée. Les lames sont ensuite rincées pendant 2 minutes dans 4 bains successifs : SSC 2X + 0,1 % SDS, SSC 1X, puis deux fois SSC 0,2X. Après séchage par centrifugation, les lames sont prêtes pour hybridation.

### Exemple 2 : Test d'hybridation utilisant la puce à ADN

Des tests d'hybridation ont été effectués avec la lame de verre préparée conformément à l'exemple 1, sur laquelle les quatre types d'oligonucléotides sont immobilisés.

Pour cela, on a déposé sous lamelle 20 µl d'une solution de l'oligonucléotide 35 mer complémentaire, marqué en position 5' par un motif CY3. La concentration en oligonucléotide complémentaire choisie a une valeur correspondant à 0,002 unité DO/µl (soit dans ce cas environ 5 µM), le solvant utilisé étant un mélange d'hybridation classique contenant du formamide, du tampon Tris EDTA pH8 [tris = tris(hydroxyméthyl)aminométhane], du SDS 10% [SDS = dodécylsulfonate de sodium] et du tampon SSC 20 X [NaCl 3M et citrate de sodium 0,3M]. L'hybridation est réalisée à 42°C pendant une nuit dans des chambres d'hybridation du type « Arrayit ». A l'issue de l'hybridation, les lames ont subi un lavage pendant 2 minutes dans 4 bains successifs : SSC 2X + 0,1 % SDS, SSC 1X, puis deux fois SSC 0,2X. Après séchage par centrifugation, la saisie des images rendant compte de la fluorescence des différents spots (tâches) a été analysée sur un scanner. L'analyse de ces images a permis de quantifier l'intensité de fluorescence des différents spots. La même procédure a été effectuée en utilisant pour l'hybridation un oligonucléotide 35 mer non complémentaire, marqué également en position 5' par un motif CY3.

Lorsque l'oligonucléotide non complémentaire est utilisé, aucune fluorescence n'est détectée. Lorsque l'oligonucléotide complémentaire est utilisé, un signal de fluorescence est observé sur tous les spots, avec les caractéristiques suivantes :
1- Bruit de fond autour des spots faible (fond noir).
2- L'intensité de fluorescence des spots correspondant à 1 et 4 est 6 fois plus faible que pour leur homologue phosphorylé en 5', pour une même concentration de dépôt. Cela montre l'effet favorable très net de la présence du groupe phosphate pour l'accrochage de l'oligonucléotide sur le support.
3- L'intensité de fluorescence des spots correspondant à 3 est 1,6 fois plus forte [puissance laser 80%, puissance photomultiplicateur 70% : intensité 48000] que pour l'oligonucléotide 2, pour une même concentration de dépôt. Cela montre l'effet favorable très net de la présence d'un groupe espaceur (11 motifs adénine) entre l'oligonucléotide et le groupe phosphate, permettant d'éloigner l'oligonucléotide de la surface du support, et facilitant ainsi l'hybridation.
4- Pour les oligonucléotides 2 et 3, les intensités de fluorescence pour des concentrations de dépôt respectivement de 50, 20 et 5 µM est de 2/2/1, ce qui montre que la concentration de dépôt optimale se situe entre 20 et 5 µM.

### Exemple 3 : Puce comportant des oligonucléotides phosphorylés en 5' porteurs de groupements espaceurs entre le groupement phosphate libre 5'-terminal et l'oligonucléotide.

On a immobilisé des oligonucléotides dans les mêmes conditions que celles décrites dans l'exemple 1, en effectuant en outre, avant l'hybridation, un traitement de la surface de la puce avec une solution de BSA (Bovine Serum Albumina : 1% BSA, 0,3% SDS dans SSC 3,5X), suivi d'un rinçage et d'un séchage, afin d'améliorer le rapport signal/bruit. L'immobilisation a été effectuée avec trois oligonucléotides 33 mer, de séquences différentes (baptisés respectivement 5, 6, 7) et phosphorylés en position terminale 5', ainsi qu'avec leurs analogues portant des espaceurs de type de type polyadénine (11 mer), polycytosine (11 mer), polyguanine (11 mer) ou polythymine (11 mer) entre le phosphate libre 5'-terminal et l'oligonucléotide, baptisés respectivement (A)₁₁-5, (A)₁₁-6, et (A)₁₁-7 ; (C)₁₁-5, (C)₁₁-6, et (C)₁₁-7 ; (G)₁₁-5, (G)₁₁-6, et (G)₁₁-7 ; (T)₁₁-5, (T)₁₁-6, et (T)₁₁-7.

Des tests d'hybridation ont été effectués avec ces oligonucléotides selon le protocole décrit dans l'exemple 2, en utilisant :
- pour l'oligonucléotide 5 et ses analogues : une solution de l'oligonucléotide 33 mer complémentaire du composé 5 et marqué en position 5' par un motif CY3 ;
- pour l'oligonucléotide 6 et ses analogues : une solution de l'oligonucléotide 33 mer complémentaire du composé 6 et marqué en position 5' par un motif CY3 ;
- pour l'oligonucléotide 7 et ses analogues : une solution de l'oligonucléotide 33 mer complémentaire du composé 7 et marqué en position 5' par un motif CY3.

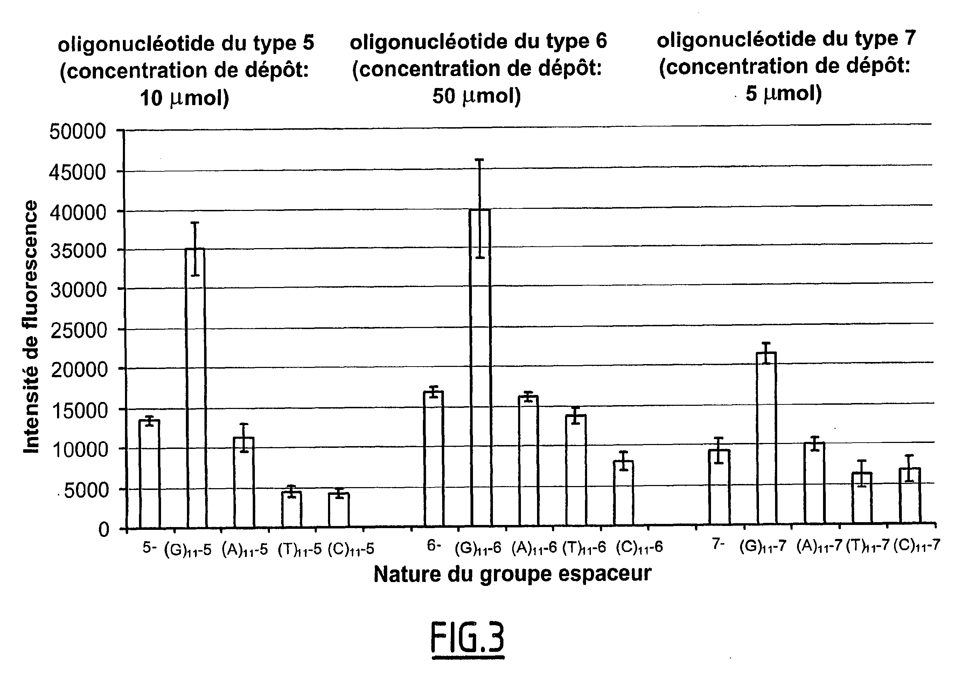

Quel que soit l'oligonucléotide 5, 6 ou 7 testé, l'intensité de fluorescence des spots correspondants aux sondes munies d'un groupe espaceur polyguanine (11 mer) [(G)₁₁-5, (G)₁₁-6, et (G)₁₁-7] est plus de deux fois plus forte que pour leurs homologues ne comportant pas de groupe espaceur ou portant un groupe espaceur polyadénine (11 mer), polycytosine (11 mer) ou polythymine (11 mer), pour une même concentration de dépôt. La figure 3 ci-annexée illustre cet effet, qui met en évidence le caractère avantageux très net de la présence d'un groupe espaceur polyguanine entre l'oligonucléotide et le groupe phosphate.

### Exemple 4 : Propriétés comparées de puces porteuses d'oligonucléotides phosphorylés en position 5' et de dispositif comportant des oligonucléotides non porteurs de groupements phosphate libre terminal.

Dans cet exemple, on a utilisé les oligonucléotides (G)₁₁-5, (G)₁₁-6 et (G)₁₁-7 de l'exemple 3 et, à titre comparatif, leurs analogues non modifiés (sans espaceur polyguanine, ni groupement phosphate libre terminal) respectivement baptisés 8, 9 et 10.

Ces oligonucléotides ont été immobilisés sur support dans les conditions de l'exemple 1. Chacun des oligonucléotides a été "spotté" à une concentration de 10 micromoles par litre, puis les lames ont été laissées pendant 24 heures dans une boite fermée.

Avant hybridation et sans rinçage préalable, les lames ont subi un traitement avec une solution de BSA, comme dans l'exemple 3. L'hybridation a ensuite été réalisée pendant 4 heures, avec les mêmes oligonucléotides complémentaires que ceux cités dans l'exemple 3, mais à une concentration de 100 nM pour chacun d'eux. Quel que soit l'oligonucléotide testé [(G)₁₁-5, (G)₁₁-6 ou (G)₁₁-7], l'intensité de fluorescence des spots qui a été mesurée est 1000 fois plus forte que celle observée pour les composés 8, 9 ou 10 analogues non modifiés, ce qui illustre notamment la spécificité de l'ancrage via le groupement phosphate libre terminal en 5'. De plus, l'intensité des spots pour les oligonucléotides (G)₁₁-5, (G)₁₁-6 et (G)₁₁-7 est conforme à celle observée dans l'exemple 3 (hybridation à 5 micromoles par litre), attestant en particulier de la bonne sensibilité de la puce.

## Revendications

1. Produit de type puce biologique, comprenant un support solide plan présentant une surface recouverte d'un métal capable de liaison de coordination avec un groupement phosphate, au moins un polymère biologique portant un groupement phosphate libre OP(O)(OH)₂ étant immobilisé sur ladite surface par liaison iono-covalente entre le groupement phosphate libre dudit polymère et le métal.

2. Produit selon la revendication 1, dans lequel le polymère biologique est un acide nucléique phosphorylé en position 5'.

3. Produit selon la revendication 1, dans lequel le polymère biologique est un acide nucléique phosphorylé en position 3'.

4. Produit selon l'une des revendications 2 ou 3, **caractérisé en ce que** l'acide nucléique présente un groupement espaceur polyguanine (polyG) entre le corps de l'acide nucléique et le groupement phosphate.

5. Produit selon la revendication 1, dans lequel le polymère biologique est une protéine phosphorylée.

6. Produit selon la revendication 1, dans lequel le polymère biologique est un oligo ou polysaccharide phosphorylé.

7. Produit selon l'une des revendications 1 à 6, dans lequel le métal est lié à la surface du support par l'intermédiaire d'une molécule espaceur.

8. Produit selon la revendication 7, dans lequel la molécule espaceur comprend une chaîne d'acide gras portant un groupement phosphonate, auquel se lie le métal par liaison iono-covalente.

9. Produit selon l'une des revendications 1 à 8, dans lequel le métal est le zirconium.

10. Produit selon la revendication 8, dans lequel la molécule espaceur est l'acide octadécylphosphonique et le métal est le zirconium.

11. Produit selon l'une des revendications 1 à 10, dans lequel le support est du verre.

12. Produit selon la revendication 1, comprenant une lame de verre présentant une surface recouverte d'une monocouche d'octadécylphosphonate de zirconium, au moins un acide nucléique portant un groupement phosphate en 5' étant immobilisé sur ladite surface par liaison iono-covalente entre ledit groupement phosphate de l'acide nucléique et le zirconium.

13. Méthode de fabrication d'un produit de type puce biologique, tel que défini dans l'une des revendications 1 à 12, comprenant l'immobilisation d'au moins un polymère biologique portant un groupement phosphate libre OP(O)(OH)₂ sur un support solide présentant une surface recouverte d'un métal capable de liaison de coordination avec un groupement phosphate, ledit polymère biologique étant immobilisé sur ladite surface par liaison iono-covalente entre le groupement phosphate libre dudit polymère et le métal.

14. Méthode selon la revendication 13, comprenant en outre une étape d'obtention du polymère biologique portant un groupement phosphate.

15. Méthode selon la revendication 14, dans laquelle le polymère est un acide nucléique phosphorylé en 5' par voie enzymatique.

16. Kit de préparation d'un produit de type puce biologique tel que défini à l'une des revendications 1 à 12, comprenant les éléments suivants :
- un support solide présentant une surface recouverte d'un métal capable de liaison de coordination avec un groupement phosphate libre OP(O)(OH)₂;
- au moins un polymère biologique portant un groupement phosphate ;
- éventuellement des réactifs.

17. Utilisation d'un produit de type puce biologique tel que défini à l'une des revendications 1 à 12, à des fins de criblage de composés susceptibles de se lier audit polymère biologique immobilisé.

18. Utilisation d'un produit de type puce biologique tel que défini à l'une des revendications 1 à 12, comme outil de diagnostic in *vitro.*

## Claims

1. A product of the biochip type, comprising a flat solid support having a surface covered with a metal capable of coordination bonding with a phosphate group, at least one biopolymer carrying a free phosphate group OP(O)(OH)₂ being immobilised on said surface by ionocovalent bonding between the free phosphate group of said polymer and the metal.

2. The product according to Claim 1, wherein the biopolymer is a nucleic acid phosphorylated in the 5' position.

3. The product according to Claim 1, wherein the biopolymer is a nucleic acid phosphorylated in the 3' position.

4. The product according to either of Claims 2 or 3, **characterised in that** the nucleic acid has a polyguanine (polyG) spacer group between the body of the nucleic acid and the phosphate group.

5. The product according to Claim 1, wherein the biopolymer is a phosphorylated protein.

6. The product according to Claim 1, wherein the biopolymer is a phosphorylated oligo- or poly-saccharide.

7. The product according to any of Claims 1 to 6, wherein the metal is bound to the surface of the support by means of a spacer molecule.

8. The product according to Claim 7, wherein the spacer molecule comprises a fatty acid chain carrying a phosphonate group to which the metal binds by ionocovalent bonding.

9. The product according to any of Claims 1 to 8, wherein the metal is zirconium.

10. The product according to Claim 8, wherein the spacer molecule is octadecylphosphonic acid and the metal is zirconium.

11. The product according to any of Claims 1 to 10, wherein the support is glass.

12. The product according to Claim 1, comprising a sheet of glass having a surface covered with a monolayer of zirconium octadecylphosphonate, at least one nucleic acid carrying a phosphate group in the 5' position being immobilised on said surface by ionocovalent bonding between said phosphate group of the nucleic acid and the zirconium.

13. A method for making a product of the biochip type, as defined in any of Claims 1 to 12, comprising the immobilisation of at least one biopolymer carrying a free phosphate group OP(O)(OH)₂ on a solid support having a surface covered with a metal capable of coordination bonding with a phosphate group, said biopolymer being immobilised on said surface by ionocovalent bonding between the free phosphate group of said polymer and the metal.

14. The method according to Claim 13, further comprising a step of obtaining the biopolymer carrying a phosphate group.

15. The method according to Claim 14, wherein the polymer is a nucleic acid phosphorylated enzymatically in the 5' position.

16. A kit for the preparation of a product of the biochip type as defined in any of Claims 1 to 12, comprising the following elements:
- a solid support having a surface covered with a metal capable of coordination bonding with a free phosphate group OP(O)(OH)₂;
- at least one biopolymer carrying a phosphate group;
- optionally reagents.

17. The use of a product of the biochip type as defined in any of Claims 1 to 12, for the purpose of screening compounds capable of binding to said immobilised biopolymer.

18. The use of a product of the biochip type as defined in any of Claims 1 to 12, as an *in vitro* diagnostic tool.

## Patentansprüche

1. Produkt vom Typ eines biologischen Chips umfassend einen planen festen Träger, der eine Oberfläche aufweist, die mit einem Metall beschichtet ist, das in der Lage ist, eine Koordinationsbindung mit einer Phosphatgruppe auszubilden, wobei mindestens ein biologisches Polymer, das eine freie Phosphatgruppe OP(O)(OH)₂ trägt, durch eine iono-kovalente Bindung zwischen der freien Phosphatgruppe des Polymers und dem Metall auf der Oberfläche immobilisiert ist.

2. Produkt nach Anspruch 1, worin das biologische Polymer eine in der 5'-Stellung phosphorylierte Nucleinsäure ist.

3. Produkt nach Anspruch 1, worin das biologische Polymer eine in der 3'-Stellung phosphorylierte Nucleinsäure ist.

4. Produkt nach einem der Ansprüche 2 oder 3, **dadurch gekennzeichnet, dass** die Nucleinsäure zwischen dem Nucleinsäureteil und der Phosphatgruppe eine Polyguanin(polyG)-Brückengruppe aufweist.

5. Produkt nach Anspruch 1, worin das biologische Polymer ein phosphoryliertes Protein ist.

6. Produkt nach Anspruch 1, worin das biologische Polymer ein phosphoryliertes Oligo- oder Polysaccharid ist.

7. Produkt nach einem der Ansprüche 1 bis 6, worin das Metall über ein Brückenmolekül an die Oberfläche des Trägers gebunden ist.

8. Produkt nach Anspruch 7, worin das Brückenmolekül eine Fettsäurekette umfasst, die eine Phosphonatgruppe aufweist, an welche das Metall durch eine iono-kovalente Bindung gebunden wird.

9. Produkt nach einem der Ansprüche 1 bis 8, worin das Metall Zirkonium ist.

10. Produkt nach Anspruch 8, worin das Brückenmolekül Octadecylphosphonsäure und das Metall Zirkonium sind.

11. Produkt nach einem der Ansprüche 1 bis 10, worin der Träger aus Glas ist.

12. Produkt nach Anspruch 1, umfassend eine Glasplatte, deren eine Oberfläche mit einer Zirkonium-Octadecylphosphonat-Monoschicht bedeckt ist, wobei mindestens eine Nucleinsäure, die eine Phosphatgruppe in der 5'-Stellung aufweist, durch eine iono-kovalente Bindung zwischen der Phosphatgruppe der Nucleinsäure und dem Zirkonium auf der Oberfläche immobilisiert ist.

13. Verfahren zur Herstellung eines Produkts vom Typ eines biologischen Chips, wie es in einem der Ansprüche 1 bis 12 definiert ist, umfassend die Immobilisierung mindestens eines biologischen Polymers, das eine freie Phosphatgruppe OP(O)(OH)₂ trägt, auf einem festen Träger, der eine Oberfläche aufweist, die mit einem Metall bedeckt ist, das in der Lage ist, eine Koordinationsbindung mit einer Phosphatgruppe auszubilden, wobei das biologische Polymer durch eine iono-kovalente Bindung zwischen der freien Phosphatgruppe des Polymers und dem Metall auf der Oberfläche immobilisiert ist.

14. Verfahren nach Anspruch 13, umfassend zusätzlich eine Stufe der Bildung eines eine Phosphatgruppe aufweisenden biologischen Polymers.

15. Verfahren nach Anspruch 14, worin das Polymer eine auf enzymatischem Wege in der 5'-Stellung phosphorylierte Nucleinsäure ist.

16. Kit zur Herstellung eines Produkts vom Typ eines biologischen Chips, wie es in einem der Ansprüche 1 bis 12 definiert ist, umfassend die folgenden Elemente:
- einen festen Träger, der eine Oberfläche aufweist, die mit einem Metall beschichtet ist, das in der Lage ist, eine Koordinationsbindung mit einer freien Phosphatgruppe OP(O)(OH)₂ auszubilden;
- mindestens ein eine Phosphatgruppe tragendes biologisches Polymer;
- eventuelle Reagentien.

17. Verwendung eines Produkts vom Typ eines biologischen Chips wie es in einem der Ansprüche 1 bis 12 definiert ist, zur Selektion von Verbindungen, die in der Lage sind, sich an das genannte immobilisierte biologische Polymer zu binden.

18. Verwendung eines Produkts vom Typ eines biologischen Chips, wie es in einem der Ansprüche 1 bis 12 definiert ist, als in vitro-Diagnosewerkzeug.
